# EUROPÄISCHE PATENTSCHRIFT

(11) **EP 2 742 003 B1**
(45) Veröffentlichungstag und Bekanntmachung des Hinweises auf die Patenterteilung: **08.07.2015**
(21) Anmeldenummer: 12745486.6
(22) Anmeldetag: 07.08.2012
(51) Int. Cl.: C02F 1/02, C02F 1/26, B01D 11/04, C07C 201/16, C02F 101/38, C02F 103/36, C02F 101/32

(54) **VERFAHREN ZUR AUFREINIGUNG VON ABWÄSSERN AUS DER AUFARBEITUNG VON ROHEN AROMATISCHEN NITROVERBINDUNGEN**
METHOD FOR PURIFYING WASTEWATER FROM REPROCESSING OF CRUDE AROMATIC NITRO COMPOUNDS
PROCÉDÉ POUR ÉPURER DES EAUX USÉES ISSUES DU TRAITEMENT DE COMPOSÉS NITROAROMATIQUES BRUTS

(30) Priorität: 09.08.2011 EP 11176904
(43) Veröffentlichungstag der Anmeldung: 18.06.2014
(73) Patentinhaber: BASF SE, 67056 Ludwigshafen (DE)
(72) Erfinder: DECKERT, Petra, 69245 Bammental (DE); DENISSEN, Leo, Brasschaat (BE); VAN DE VOORDE, Bart, B-2060 Antwerpen (BE); LESCHINSKI, Julia, B-1050 Ixelles (Bruxelles) (BE); DEIBEL, Stefan, Robert, B-1560 Hoeilaart (BE); FANKHÄNEL, Matthias, 67273 Dackenheim (DE); NETO, Samuel, B-1050 Bruxelles (BE)
(86) Internationale Anmeldenummer: PCT/EP2012/065381
(87) Internationale Veröffentlichungsnummer: WO 2013/020961

(56) Entgegenhaltungen:
- WO-A1-2011/021057

## Beschreibung

Die Erfindung betrifft ein Verfahren zur Aufreinigung von rohen aromatischen Nitroverbindungen, die aus der Nitrierung von aromatischen Verbindungen stammen, umfassend die ein- oder mehrfache Durchführung der folgenden Waschstufe (a):
(a) Inkontaktbringen der rohen aromatischen Nitroverbindung (N-ein) mit einer wässrigen Phase (W-ein) und anschließende Phasentrennung unter Erhalt einer organischen Phase (N-res) und einer wässrigen Phase (W-res),
   wobei in einer oder mehreren der Waschstufen (a) mindestens ein Demulgator (D) zugegen ist, der eine amphiphile Verbindung ist.

Aromatische Nitroverbindungen, insbesondere Mononitrobenzol, werden in kommerziellen Verfahren üblicherweise durch direkte Nitrierung von Benzol mit einer Mischung aus Salpetersäure und Schwefelsäure, der sogenannten Nitriersäure, hergestellt. Bei dieser Reaktion handelt es sich um eine Zweiphasenreaktion, deren Reaktionsgeschwindigkeit durch den Massentransfer zwischen den Phasen und durch die chemische Kinetik determiniert wird. Von besonderer industrieller Bedeutung sind kontinuierliche Verfahren, wobei die adiabatische Reaktionsführung in jüngerer Zeit besondere Bedeutung erlangt hat.

Das aus der Nitrierung der aromatischen Ausgangsverbindung erhaltene Reaktionsprodukt (Rohprodukt), insbesondere Mononitrobenzol, fällt zunächst als zweiphasiges Gemisch an, wobei die organische Phase neben der organischen Nitroverbindung weitere organische Nebenprodukte und nicht umgesetzte organische Ausgangsstoffe enthält. Die wässrige Phase umfasst neben organischen Bestandteilen wie zum Beispiel Mononitrobenzol und Benzol natürlich unverbrauchte Nitriersäure. Die säurehaltige wässrige Phase wird nach dem Stand der Technik üblicherweise in einem Säurekonzentrator (sulfuric acid concentration, SAC) aufkonzentriert und wieder in die Nitrierungsreaktion zurückgeführt.

Die aus der Nitrierung erhaltene organische Phase, nachfolgend als rohe aromatische Nitroverbindung (N-ein) bezeichnet, ist sowohl mit organischen Nebenkomponenten wie z. B. Dinitrobenzol, Benzol, Nitrophenolen als auch mit Nitriersäure verunreinigt und bedarf einer mehrstufigen Aufarbeitung, die hohen Anforderungen hinsichtlich Energie- und Prozesskosten, Ausbeute und Aufreinigung von Abfallströmen nach umwelttechnischen Gesichtspunkten genügen muss. Verfahren zur Aufarbeitung von rohen aromatischen Nitroverbindungen sind aus dem Stand der Technik bekannt.

Üblicherweise schließt sich an die Abtrennung der rohen aromatischen Nitroverbindung, insbesondere rohes Mononitrobenzol, von der säurehaltigen wässrigen Phase mindestens ein Waschvorgang der rohen aromatischen Nitroverbindung mit Wasser oder einer wässrigen Lösung an.

Die wässrige Phase, welche aus dem vorgenannten Waschvorgang resultiert (nachfolgend als Abwasser bezeichnet), umfasst neben Wasser und Salzen noch organische Verbindungen wie Mononitrobenzol, Dinitrobenzol, Nitrophenole (mono- und polynitrierte Phenole) und Benzol.

Die erforderliche Abreicherung der unerwünschten organischen Bestandteile aus dem Abwasser vor dessen Einleitung in eine biologische Abwasseraufbereitung (Kläranlage) stellt einen signifikanten Anteil der Investitionskosten bei der Installation von Anlagen zur Herstellung von aromatischen Nitroverbindungen dar.

Die EP 1 593 654 A1 beschreibt ein Verfahren zur Aufarbeitung von alkalischen Abwässern, die bei der Wäsche von rohem Nitrobenzol entstehen, wobei das rohe Nitrobenzol durch adiabate Nitrierung von Benzol mit Nitriersäure hergestellt und anschließend in einer sauren Wäsche und danach in einer alkalischen Wäsche gewaschen wird, wobei ein alkalisches Abwasser enthaltend Benzol in Konzentrationen von 100 bis 3000 ppm und Nitrobenzol in Konzentrationen von 1000 bis 10000 ppm erhalten wird. Aus dem alkalischen Abwasser wird anschließend nicht gelöst vorliegendes Benzol und/oder Nitrobenzol abgeschieden, und optional wird aus dem alkalischen Abwasser anschließend restliches Benzol und/oder Nitrobenzol durch Strippen entfernt. Anschließend wird das alkalische Abwasser unter Ausschluss von Sauerstoff auf Temperaturen von 150 bis 500 °C unter Überdruck erhitzt. Saure Abwässer gemäß dem Stand der Technik sind jedoch in Bezug auf die weitere Aufarbeitung nachteilig. In der Regel enthalten sie Bestandteile, die vor der Zuführung in eine biologische Abwasseraufarbeitung unter zusätzlichem Aufwand zu entfernen sind. Saure Abwässer führen oftmals zudem zu Korrosionsproblemen bei nachfolgenden Aufreinigungsstufen, die nur durch zusätzlichen technischen Aufwand zu beheben sind.

Nach dem Stand der Technik erhaltenes Abwasser enthält zudem in allen Stufen Nitrophenolate bzw. Nitrophenole, so dass alle Abwässer einer aufwendigen und teuren thermolytischen Zersetzung zugeführt werden müssen. Es ist somit wünschenswert, ein Verfahren zur Aufreinigung von rohen aromatischen Nitroverbindungen zur Verfügung zu stellen, welches höchstens ein Teil der Abwässer einer thermolytischen Wasseraufarbeitung zuführt.

Die Abtrennung der organischen Bestandteile aus dem Abwasser kann beispielsweise durch eine ggf. mehrstufige Extraktion mit Benzol erfolgen. Ein bekanntes Verfahren ist die ggf. mehrstufige Strippung der organischen Bestandteile mit Wasserdampf, wobei insbesondere leichtsiedende organische Verunreinigungen entfernt werden, und anschließende thermolytische oder oxidative Zersetzung noch vorhandener organischer Bestandteile im resultierenden Abwasser.

So beschreibt die EP 0 953 546 A2 ein Verfahren zum Abbau von aromatischen Nitroverbindungen in Abwässern durch Erhitzung des Abwassers auf Temperaturen von 150 bis 350 °C unter einem Druck von 10 bis 300 bar. Die EP 0 953 546 A2 führt aus, dass die so behandelten Abwässer biologisch gereinigt werden können.

Durch die Zersetzung von Nitroverbindungen in wässrigen Abwässern nach dem Stand der Technik entsteht jedoch Ammoniak, der sich in biologischen Kläranlagen nachteilig auswirkt. Einer biologischen Abwasseraufbereitung zugeführtes NH₃-haltiges Abwasser muss zunächst einer Nitrifikation unterzogen werden. Als Nitrifikation bezeichnet man die bakterielle Oxidation von Ammoniak (NH₃) zu Nitrat (NO₃⁻). Sie besteht aus zwei gekoppelten Teilprozessen: Im ersten Teil wird Ammoniak zu Nitrit oxidiert, das im zweiten Teilprozess zu Nitrat oxidiert wird. Die Nitrifikation ist mit einer Produktion von Säure (H⁺-Bildung) verbunden, der pH-Wert wird abgesenkt, wenn die gebildete Säure nicht neutralisiert wird, etwa durch Umsetzung mit Calciumcarbonat (CaCO₃). Die gebildete Säure belastet die Pufferkapazität des Wassers und kann das Wasser bzw. den Boden versäuern. Da nitrifizierende Mikroorganismen nur im neutralen bis leicht alkalischen Bereich stoffwechseln, kann durch die Versäuerung die vollständige Umwandlung des fischtoxischen Ammoniums/Ammoniak in Abwasser-Kläranlagen verhindert werden (Autoinhibition).

Das gebildete Nitrat wird anschließend in einer weiteren Stufe der biologischen Abwasserbehandlung einer Denitrifikation unter Bildung von N₂ unterzogen. Unter Denitrifikation versteht man die Umwandlung des im Nitrat (NO₃⁻) gebundenen Stickstoffs zu molekularem Stickstoff (N₂) durch bestimmte heterotrophe und einige autotrophe Bakterien, die danach als Denitrifikanten bezeichnet werden.

Demzufolge kann das nach dem Stand der Technik entstehende Abwasser erst nach einer aufwendigen Aufarbeitung unter Entfernung aller Nitrophenole der biologischen Abwasserbehandlung zugeführt werden.

Darüber hinaus sind die aus dem Stand der Technik bekannten Waschstufen nicht zufriedenstellend: die Phasentrennung erfolgt nicht ausreichend schnell und/oder nicht vollständig, so dass unerwünschte Bestandteile in der jeweiligen Phase verbleiben.

Die WO2011/021057 offenbart ein Verfahren zur Aufreinigung von rohen aromatischen Nitroverbindungen, die aus der Nitrierung von aromatischen Verbindungen stammen, umfassend die ein- oder mehrfache Durchführung der folgenden Waschstufe (a):
(a) Inkontaktbringen der rohen aromatischen Nitroverbindung mit einer wässrigen Phase und anschließende Phasentrennung unter Erhalt einer organischen Phase und einer wässrigen Phase, wobei in einer oder mehreren der Waschstufen (a) mindestens ein Demulgator (D) zugegen ist, der eine anorganische Säure ist.

Aufgabe der vorliegende Erfindung war, ein Verfahren zur Aufreinigung von Rohprodukten aus der Nitrierung von aromatischen Verbindungen bereitzustellen, welches die vorgenannten Nachteile nicht oder in geringerem Umfang aufweist. Insbesondere sollten die Waschstufen möglichst schnell durchführbar sein. Die Phasentrennung sollte in gegebener Zeit möglichst vollständig sein.

Das Verfahren sollte außerdem zumindest teilweise Abwässer erzeugen, die gegebenenfalls nach Strippung und optional Entfernung von Ammoniak unmittelbar einer biologischen Abwasseraufbereitung zugeführt werden können, und zwar unter Auslassung einer thermischen und/oder oxidativen Aufarbeitung. Die insgesamt produzierte Menge an Abwasser sollte möglichst gering sein.

Die vorgenannten Aufgaben werden gelöst durch das erfindungsgemäße Verfahren. Bevorzugte Ausführungsformen sind den Ansprüchen und der nachfolgenden Beschreibung zu entnehmen. Kombinationen bevorzugter Ausführungsformen verlassen den Rahmen der vorliegenden Erfindung nicht, insbesondere, was Kombinationen bevorzugter Ausführungsformen der unterschiedlichen Stufen des erfindungsgemäßen Verfahrens angeht.

Erfindungsgemäß umfasst das Verfahren die ein- oder mehrfache Durchführung der folgenden Waschstufe (a):
(a) Inkontaktbringen der rohen aromatischen Nitroverbindung (N-ein) mit einer wässrigen Phase (W-ein) und anschließende Phasentrennung unter Erhalt einer organischen Phase (N-res) und einer wässrigen Phase (W-res),
   wobei in einer oder mehreren der Waschstufen (a) mindestens ein Demulgator (D) zugegen ist, der eine amphiphile Verbindung ist.

Der Begriff "Waschen" beziehungsweise "Waschstufe" kennzeichnet im Rahmen der vorliegenden Erfindung das Inkontaktbringen einer organischen Phase mit einer wässrigen Phase, wobei mindestens ein Bestandteil der organischen Phase zumindest teilweise in die wässrige Phase übergeht, einschließlich der anschließenden Phasentrennung. Das Waschen organischer Phasen und die anschließende Abtrennung der Phasen ist dem Fachmann an sich bekannt und kann in an sich bekannten Apparaturen wie Misch-Abtrenneinheiten (Mischeinheit gefolgt von Abtrenneinheit) oder Extraktoren wie z. B. Extraktionskolonnen erfolgen.

Unter mehrfacher Durchführung (synonym: mehrstufige Durchführung) wird verstanden, dass sich jede aufeinander folgende Stufe in der eingesetzten wässrigen Phase (W-ein) von der vorhergehenden Stufe unterscheidet. Es ist darüber hinaus auch möglich, jede einzelne Stufe in einem Schritt oder in mehreren Sub-Schritten (Wiederholungen) durchzuführen. Unter Durchführung einer Stufe in mehreren Sub-Schritten ist zu verstehen, dass die Abfolge Inkontaktbringen - Phasentrennung mehrfach hintereinander mit einer wässrigen Phase, z. B. W1-ein, durchgeführt wird. Hierbei ist bevorzugt, die eingesetzte wässrige Phase im Gegenstrom zum Strom der rohen aromatischen Nitroverbindung N-ein zu führen, d. h. bei einer Durchführung in zwei Sub-Schritten wird die beim zweiten Sub-Schritt resultierende wässrige Phase im ersten Sub-Schritt mit der eingesetzten rohen aromatischen Nitroverbindung N1-ein in Kontakt gebracht. Dies wird weiter unten näher erläutert.

Bei einer mehrfachen Durchführung der Waschstufe (a), was bevorzugt ist, werden diese Stufen in der Reihenfolge ihrer Durchführung nachfolgend als Waschstufen (a1), (a2) usw. bezeichnet. Die Waschstufe umfassend das Inkontaktbringen der rohen aromatischen Nitroverbindung (N-ein) mit einer wässrigen Phase (W-res) und anschließende Phasentrennung unter Erhalt einer organischen Phase (N-res) und einer wässrigen Phase (W-res) wird entsprechend mehrfach durchgeführt; die entsprechenden Phasen werden als W1-ein, W2-ein, W1-res, W2-res, N1-ein, N2-ein, N2-ein, N2-res usw. bezeichnet.

Erfindungsgemäß ist in mindestens einer Waschstufe (a) mindestens ein Demulgator zugegen. Im Falle einer zweistufigen Durchführung bedeutet dies, dass in Stufe (a1) oder in Stufe (a2) oder in beiden Stufen (a1) und (a2) mindestens ein Demulgator (D) zugegen ist.

Sofern in mehren Stufen, beispielsweise in Stufe (a1) und in Stufe (a2), ein Demulgator (D) verwendet wird, dann können diese gleich oder unterschiedlich sein. Vorzugsweise wird bei einer mehrstufigen Durchführung, insbesondere bei einer Durchführung in zwei Stufen, in Stufe (a1) der gleiche Demulgator oder die gleichen Demulgatoren verwendet wie in Stufe (a2).

Der Begriff "Demulgator" wird im Rahmen der vorliegenden Erfindung gleichbedeutend mit "Phasentrennmittel" verwendet und kennzeichnet ein Hilfsmittel, welches die Trennung der organischen Phase und der wässrigen Phase beschleunigt und/oder die Güte der Phasentrennung verbessert. Ohne sich beschränken zu wollen besteht die Vorstellung, dass die Demulgatoren als grenzflächenaktive Substanz wirken und so die Geschwindigkeit der Ausbildung einer veränderten, d.h. verringerten Grenzfläche zwischen wässriger und organischer Phase beeinflussen. Demulgatoren werden im Stand der Technik auch als Emulsionsspalter oder Emulsionstrennmittel bezeichnet. Wenngleich aus dem Stand der Technik bekannte Demulgatoren grundsätzlich auch für die vorliegende Erfindung in Betracht kommen, bedeutet dies nicht, dass die wässrigen Phasen und organische Phasen in allen Fällen eine (stabile) Emulsion bilden. Der Begriff "in Gegenwart mindestens eines Demulgators" bedeutet, dass in mindestens einem Sub-Schritt der jeweiligen Stufe, beispielsweise Stufe (a1) und/oder (a2), mindestens ein Demulgator zugegen ist. Es ist bevorzugt, wenn der Demulgator bei der Durchmischung der organischen Phase (N-ein) und der wässrigen Phase (W-ein) in der jeweilige Stufe zugegen ist, da sich so eine gute Verteilung an der Grenzfläche zwischen den beiden Phasen einstellen lässt. Sofern in der jeweiligen Stufe bzw. in dem jeweiligen Sub-Schritt (Inkontaktbringen - Phasentrennung) eine Mischapparatur und anschließend eine Phasentrennapparatur verwendet wird, so erfolgt eine Zugabe des Demulgators bzw. der Demulgatoren somit vorzugsweise in der Mischapparatur, da die Phasentrennapparatur alleine keine ausreichende Durchmischung bietet.

Als Demulgator (D) werden amphiphile Verbindungen eingesetzt. Amphiphile Verbindungen sind Verbindungen mit mindestens einem hydrophilen und mindestens einem hydrophoben molekularen Abschnitt, wobei ein Abschnitt eine funktionelle Gruppe, ein Comonomer, eine Endgruppe oder ein Block eines Blockcopolymers sein kann. Entsprechende amphiphile Verbindungen sind dem Fachmann bekannt. Geeignete Demulgatoren (D) können unterschiedliche Funktionsweisen aufweisen. Insbesondere können die Demulgatoren (D) die umgekehrte Dispergierrichtung stabilisieren, Benetzungseigenschaften von Feststoffen verändern oder andere Verbindungen, die als Emulgatoren fungieren, aus der Grenzfläche verdrängen, aber weniger stabilisierend wirken als diese.

Die Demulgatoren (D) werden im Rahmen der vorliegenden Erfindung vorzugsweise in einer Menge von 0,1 bis 1000 ppm, insbesondere von 1 bis 200 ppm, besonders bevorzugt von 1 bis 100 ppm, ganz besonders bevorzugt von 2 bis 60 ppm eingesetzt, jeweils bezogen auf die gesamte Gewichtsmenge der in der jeweiligen Stufe eingesetzten wässrigen Phase. Im Falle einer zweistufigen Durchführung bezieht sich die Menge des Demulgators in ppm auf die Gewichtsmenge W1-ein und/oder W2-ein. Die Gewichtsmenge des Demulgators wird hierbei der gesamten Gewichtsmenge der eingesetzten wässrigen Phase W-ein zugezählt.

Amphiphile anionische Copolymere sind als Demulgator (D) bevorzugt. Der Gehalt an Monomereinheiten, die anionische Gruppen enthalten, beträgt vorzugsweise von 0,1 bis 5 Gew.-% bezogen auf die Gewichtsmenge des Copolymers. Als anionische Gruppen werden vorzugsweise Carboxylatgruppen verwendet.

Besonders bevorzugt sind die Demulgatoren (D) im Wesentlichen wasserlöslich, insbesondere wasserlöslich. Im Wesentlichen wasserlöslich sind Demulgatoren (D) dann, wenn sie entweder vollständig wasserlöslich sind oder bei 10 Gew.-%igem Anteil in destilliertem Wasser eine höchstens geringfügige Trübung aufweisen. Durch Verwendung wasserlöslicher Demulgatoren wird verhindert, dass diese in das Produkt gelangen. Vielmehr werden die Demulgatoren dann im Rahmen des erfindungsgemäßen Verfahrens mit der wässrigen Phase abgetrennt.

Bevorzugte amphiphile anionische Copolymere weisen ein zahlenmittleres Molekulargewicht von 5.000 bis 20.000 g/mol auf. Bevorzugte amphiphile anionische Copolymere weisen außerdem ein K-Wert nach ISO 1628-1 (1Gew.-% Trockensubstanz in destilliertem Wasser) von 25 bis 50 auf.

Entsprechende amphiphile anionische Copolymere sind dem Fachmann an sich bekannt. Sie sind insbesondere durch Copolymerisation von Acrylsäure und/oder Maleinsäure mit hydrophilen Monomereinheiten, beispielsweise Vinylmonomere oder Olefine, erhältlich.

Die Auswahl geeigneter Demulgatoren (D) trifft der Fachmann anhand des folgenden Eigenschaftsprofils: geeignete Demulgatoren sind in geringen Konzentrationen wirksam, insbesondere im Bereich von 1 bis 100 ppm; sie sind chemisch inert; sie stören nicht im Produkt, beispielsweise weil sie nicht abwasserrelevant sind, oder gelangen dort nicht hin.

Copolymere enthaltend Maleinsäureinheiten, insbesondere Copolymere auf Basis von Maleinsäure und mindestens einem Olefin, sind als Demulgator (D) besonders bevorzugt.

Das vorliegende Verfahren ist insbesondere für die Aufarbeitung von Abwässern geeignet, welche bei der Aufreinigung von rohem Mononitrobenzol entstehen, welches durch Nitrierung von Benzol erhalten wurde. Aus diesem Grund wird das Verfahren exemplarisch anhand dieser speziellen Aufreinigung erläutert. Der Fachmann kann jedoch die genannten Ausführungsformen ohne Schwierigkeiten auf andere aromatische Ausgangsverbindungen als Benzol beziehungsweise auf andere Produkte als Mononitrobenzol übertragen.

Die rohe aromatische Nitroverbindungen, die mit dem erfindungsgemäßen Verfahren aufgereinigt werden können, stammen vorzugsweise aus Nitrierungsanlagen zur Nitrierung von aromatischen Verbindungen wie z.B. Nitrobenzolanlagen, Dinitrotoluolanlagen und Nitrotoluol- und Nitroxylolanlagen. Vorzugsweise ist die rohe aromatische Nitroverbindung rohes Mononitrobenzol, welches durch Nitrierung von Benzol erhalten wird.

Die Nitrierung von aromatischen Ausgangsverbindungen (insbesondere Benzol) zu aromatischen Nitroverbindungen (insbesondere Mononitrobenzol) kann nach den aus dem Stand der Technik bekannten Verfahren erfolgen. Geeignete Verfahren werden beispielsweise in der EP 043 6 443 A2 und in Kirk-Othmer, Encyclopedia of Chemical Technology, "Nitrobenzene and Nitrotoluenes", online veröffentlicht am 14.10.2005 beschrieben.

Nachdem die flüssigen Produkte den Nitrierungsreaktor verlassen haben, wird zunächst eine Phasentrennung vorgenommen, bei der eine organische Phase anfällt, die hauptsächlich Mononitrobenzol sowie nicht-umgesetztes Benzol und Spuren von organischen Nebenkomponenten, insbesondere Nitrophenole, enthält. Die wässrige Phase enthält im Wesentlichen Wasser und Schwefelsäure. Nach der Abtrennung des Reaktionswassers wird üblicherweise die wässrige Phase dem Prozess erneut zugeführt, während die organische Wertproduktphase (die rohe aromatische Nitroverbindung) erfindungsgemäß behandelt wird. Hierdurch werden insbesondere Nitrophenole entfernt.

In einer bevorzugten Ausführungsform umfasst das Verfahren zur Aufreinigung von rohen aromatischen Nitroverbindungen, die aus der Nitrierung von aromatischen Verbindungen stammen, die folgenden Waschstufen (a1) und (a2), wobei jede der Stufen (a1) und (a2) ein- oder mehrfach hintereinander durchgeführt werden kann:
(a1) Inkontaktbringen der rohen aromatischen Nitroverbindung (N1-ein) mit einer ersten wässrigen Phase (W1-ein) umfassend mindestens eine Base (B) und anschließende Phasentrennung unter Erhalt einer organischen Phase (N1-res) und einer wässrigen Phase (W1-res); und anschließend
(a2) Inkontaktbringen der in Stufe (a1) erhaltenen organischen Phase (N1-res) mit einer zweiten wässrigen Phase (W2-ein) und anschließende Phasentrennung unter Erhalt einer aufgereinigten organischen Phase (N2-res) und mindestens einer wässrigen Phase (W2-res), wobei die eingesetzte wässrige Phase (W2-ein) einen pH-Wert von 6 bis 9 aufweist.

Die Waschstufen (a1) und (a2) der bevorzugten Ausführungsform werden im Folgenden näher erläutert. Es ist dem Fachmann jedoch klar, dass er die nachfolgend erläuterten bevorzugten Ausführungsformen auch auf Ausführungsformen mit weniger oder mehr als zwei Waschstufen, z. B. drei, anwenden kann.

### Waschstufe (a1)

Die nachfolgend erläuterte Waschstufe (a1) wird auch als alkalische Wäsche bezeichnet. Die in Stufe (a1) eingesetzte rohe aromatische Nitroverbindung N1-ein wird auch als organische Phase N1-ein bezeichnet. Die Waschstufe (a1) kann erfindungsgemäß mehrfach hintereinander durchgeführt werden.

Im Rahmen dieser bevorzugten Ausführungsform erfolgt gemäß Waschstufe (a1) ein Inkontaktbringen der rohen aromatischen Nitroverbindung (N1-ein) mit einer ersten wässrigen Phase (W1-ein), die mindestens eine Base (B) umfasst, und anschließende Phasentrennung unter Erhalt einer organischen Phase (N1-res) und einer wässrigen Phase (W1-res).

Vorrichtungen zur Durchführung von Stufe (a1) sind dem Fachmann bekannt. Vorzugsweise wird in einer ersten Vorrichtung, einer Mischvorrichtung, zunächst eine intensive Durchmischung der beiden Phasen durchgeführt, um die Bildung von Phenolaten und den Übergang von Phenolaten aus der organischen Phase (N1-ein) in die wässrige Phase (W1-ein) zu beschleunigen. Es hat sich als vorteilhaft erwiesen, die Durchmischung unter Rühren durchzuführen. Anschließend erfolgt vorzugsweise in einer zweiten Vorrichtung, einer Phasentrennapparatur, die Trennung der beiden Phasen. Es ist jedoch auch möglich, die beiden genannten Vorrichtungen in einer Vorrichtung zu vereinen. Als Phasentrennapparatur kommen im Prinzip alle dem Fachmann hierfür bekannten Apparaturen in Betracht, insbesondere Settler und Zentrifugen.

Der pH-Wert der ersten wässrigen Phase (W1-ein) enthaltend mindestens eine Base (B) beträgt in der eingesetzten Form vorzugsweise von 9 bis 14, besonders bevorzugt von 9 bis 12,5, insbesondere von 9,5 bis 11

Geeignete Basen (B) sind insbesondere Ammoniak und Alkalimetallhydroxide. Alkalimetallhydroxide sind bevorzugt, insbesondere Lithiumhydroxid, Natriumhydroxid, Kaliumhydroxid und/oder Rubidiumhydroxid. Natriumhydroxid ist als Base (B) besonders bevorzugt.

Die Zugabe der Base (B) erfolgt vorzugsweise in Form von wässrigen Lösungen der Base, d.h. zumindest Teile der in Stufe (a) eingesetzten wässrigen Lösung enthalten die genannte Base (B). Die vorzugsweise im Überschuss zugesetzte Base (B) erzeugt Phenolate aus den phenolischen organischen Verbindungen und neutralisiert die in der organischen Phase befindliche Nitriersäure unter Bildung von löslichen Salzen. Der resultierende pH-Wert des Abwassers (W1-res) beträgt vorzugsweise von 8 bis 14, besonders bevorzugt 9 bis 13, insbesondere 9 bis 11

Wie bereits oben ausgeführt, kann die Waschstufe (a1) mehrfach hintereinander ausgeführt werden, vorzugsweise in zwei oder drei Sub-Schritten (Wiederholungen), vorzugsweise in einer Sequenz von Misch- und Phasentrennvorrichtungen. Dabei ist es vorteilhaft, die wässrige Phase (W1-ein) im Rahmen der Waschstufe (a1) im Gegenstrom zur organischen Phase (N1-ein) zu führen. Hierdurch kommt vorgereinigte organische Phase mit wässriger Phase (W1-ein) in Kontakt, die noch keinen Sub-Schritt durchlaufen hat. Hierdurch können die Phenolate besonders vollständig extrahiert und in die wässrige Phase überführt werden.

Grundsätzlich kommen zahlreiche wässrige Phasen (W1-ein) in Betracht, sofern sie den Anforderungen in Bezug auf Base (B) genügen. Vorzugsweise werden nitrophenolhaltige Abwässer aus der Aufarbeitung der Nitriersäure als wässrige Phase verwendet. Hierdurch ist es möglich, verschiedene nitrophenolhaltige Abwässer zu vereinen und so die Menge an Abwasser, aus dem Nitrophenole durch Thermolyse oder Ozonolyse entfernt werden müssen, zu minimieren.

Das aus Stufe (a1) resultierende Abwasser enthält üblicherweise neben Wasser noch Restmengen an Benzol und Nitrobenzol sowie Nitrophenole. Das aus Stufe (a) resultierende Abwasser enthält typischerweise Benzol in Konzentrationen von 10 bis 3000 ppm, vorzugsweise von 100 bis 1000 ppm und Nitrobenzol in Konzentrationen von 500 bis 10000 ppm, vorzugsweise von 1200 bis 8000 ppm. Das Abwasser enthält ferner üblicherweise Nitrophenolate in einer Konzentration von 1000 bis 20000 ppm, insbesondere 2000 bis 8000 ppm. Die Einheit ppm bezieht sich im Rahmen der vorliegenden Erfindung grundsätzlich auf Gewichtsanteile.

Insbesondere seien folgende Nitrophenole, die auch in Form ihrer wasserlöslichen Salze vorliegen können, genannt: Mono-, Di- und Trinitrophenole, Mono-, Di- und Trinitrokresole, Mono-, Di- und Trinitroresorcine, Mono-, Di-und Trixylenole.

### Waschstufe (a2)

Im Rahmen der bevorzugten Ausführungsform erfolgt gemäß Waschstufe (a2) anschließend ein Inkontaktbringen der in Stufe (a1) erhaltenen organischen Phase (N1-res) mit einer zweiten wässrigen Phase (W2-ein) und anschließende Phasentrennung unter Erhalt einer aufgereinigten organischen Phase (N2-res) und mindestens einer wässrigen Phase (W2-res), wobei die eingesetzte wässrige Phase (W2-ein) einen pH-Wert von 6 bis 9 aufweist. Vorzugsweise wird Stufe (a2) in Abwesenheit einer Base (B) in der wässrigen Phase (W2-ein) durchgeführt. Diese ein- oder mehrfach sequentiell wiederholte Waschstufe (a2) wird auch als neutrale Wäsche bezeichnet.

Vorrichtungen zur Durchführung von Stufe (a2) sind dem Fachmann bekannt. Es können die gleichen Vorrichtungen wie bei Stufe (a1) verwendet werden. Vorzugsweise wird in einer ersten Vorrichtung, einer Mischvorrichtung, zunächst eine intensive Durchmischung der beiden Phasen durchgeführt, um den Übergang von Salzen von der organischen Phase (N2-ein) in die wässrige Phase (W2-ein) zu beschleunigen. Es hat sich als vorteilhaft erwiesen, die Durchmischung durch Rühren zu erzeugen. Anschließend erfolgt vorzugsweise in einer zweiten Vorrichtung, einer Phasentrennapparatur, die Trennung der beiden Phasen. Es ist jedoch auch möglich, die beiden genannten Vorrichtungen in einer Vorrichtung zu vereinen.

Wie bereits oben ausgeführt, kann die Waschstufe (a2) mehrfach hintereinander ausgeführt werden, vorzugsweise in ein oder zwei Sub-Schritten (Wiederholungen), vorzugsweise in einer Sequenz von Misch- und Phasentrennvorrichtungen. Dabei ist es vorteilhaft, die wässrige Phase (W2-ein) im Rahmen der Waschstufe (a2) im Gegenstrom zur organischen Phase (N2-ein) zu führen. Hierdurch kommt die aufgereinigte organische Phase (N2-res) mit wässriger Phase in Kontakt, die noch keinen Sub-Schritt durchlaufen hat. Hierdurch können anorganische Salze besonders vollständig extrahiert und in die wässrige Phase überführt werden.

In einer bevorzugten Ausführungsform wird die Stufe (a2) in zwei Sub-Schritten (Wiederholungen) durchgeführt und die dabei eingesetzte wässrige Phase (W2-ein) im Rahmen der Waschstufe (a2) im Gegenstrom zur organischen Phase (N2-ein) geführt.

Grundsätzlich kommen zahlreiche wässrige Phasen (W2-ein) in Betracht, sofern sie den erfindungsgemäßen Anforderungen genügen. Vorzugsweise werden Abwässer verwendet, die im Wesentlichen frei von Nitrophenolen sind. Als im Wesentlichen frei von Nitrophenolen wir eine wässrige Phase dann bezeichnet, wenn sie höchstens 30 ppm, insbesondere höchstens 20 ppm Nitrophenole enthält. Besonders bevorzugt weist die wässrige Phase W2-ein ein Gehalt an Nitrophenolen von höchstens 5 ppm auf, insbesondere höchstens 3 ppm. Entsprechend werden bevorzugt Abwässer aus einem anderen Herstellungsprozess verwendet, die im Wesentlichen frei von Nitrophenolen sind. Vorzugsweise wird als wässrige Phase (W2-ein) ein Abwasser verwendet, welches aus der Hydrierung von Mononitrobenzol zur Anilinherstellung resultiert. Hierdurch ist es möglich, mehrere Abwässer aus unterschiedlichen Herstellungsprozessen gemeinsam und somit kostengünstig aufzuarbeiten. Die Abwassermenge in einem kombinierten Prozess wird dabei insgesamt reduziert.

In einer bevorzugten Ausführungsform, die unabhängig von der vorgenannten bevorzugten Ausführungsformen realisiert werden kann, wird in Anschluss an Stufe (a) und vor Durchführung von Stufe (b) beziehungsweise Stufe (c) aus dem Abwasser noch enthaltenes, nicht gelöst vorliegendes Benzol und/oder Nitrobenzol abgetrennt.

Die Abtrennung des nicht gelöst vorliegenden Nitrobenzols kann dabei durch Abscheider, Absetzbehälter oder andere Phasentrennapparaturen erfolgen. Bevorzugt wird ein Absetzbehälter eingesetzt. Die genannte Abtrennung kann alternativ in Form einer Extraktion durchgeführt werden, wie sie in der WO 2009/027416 beschrieben wird. Das so abgetrennte Benzol und/oder Nitrobenzol wird dann bevorzugt wieder dem Nitrierprozess bzw. dem rohen Nitrobenzol zugeführt.

Die aus Waschstufe (a2) resultierende wässrige Phase W2-res weist vorzugsweise einen Gehalt an Nitrophenolen von höchstens 20 ppm auf, insbesondere von 0,001 bis 20 ppm. Besonders bevorzugt beträgt der Gehalt an Nitrophenolen in W2-res höchstens 10 ppm, insbesondere von 0,001 bis 10 ppm, ganz besonders bevorzugt höchstens 5 ppm, insbesondere von 0,001 bis 5 ppm.

In einer bevorzugten Ausführungsform umfasst das erfindungsgemäße Verfahren weitere Stufen der Aufarbeitung der aus dem Verfahren resultierenden wässrigen Phasen W1-res und W2-res.

In einer bevorzugten Ausführungsform, die unabhängig von den vorgenannten bevorzugten Ausführungsformen realisiert werden kann, wird die wässrige Phase (W1-res) folgenden weiteren Aufarbeitungsstufen unterzogen:
(b) optional Entfernung organischer Bestandteile aus mindestens einem Teil der in Stufe (a) erhaltenen wässrigen Phase (W1-res) durch Strippung vorzugsweise mit Wasserdampf,
(c) Entfernung organischer Verbindungen aus mindestens einem Teil der aus Stufe (a) beziehungsweise Stufe (b) resultierenden wässrigen Phase (W1-res) durch thermischen und/oder oxidativen Abbau,
(d) destillative Abreicherung von Ammoniak aus zumindest einem Teil der aus Stufe (c) resultierenden wässrigen Phase, und
(e) optional Zuführung zumindest eines Teiles der aus Stufe (d) resultierenden wässrigen Phase zu einer biologischen Abwasseraufbereitung.

In einer anderen bevorzugten Ausführungsform, die unabhängig von der vorgenannten bevorzugten Ausführungsform realisiert werden kann, sofern die resultierenden wässrigen Phasen nicht vereinigt werden, wird die wässrige Phase (W2-res) folgenden weiteren Aufarbeitungsstufen unterzogen:
(b) optional Entfernung organischer Bestandteile aus mindestens einem Teil der in Stufe (a) erhaltenen wässrigen Phase (W2-res) durch Strippung vorzugsweise mit Wasserdampf,
(d) optional destillative Abreicherung von Ammoniak aus zumindest einem Teil der aus Stufe (c) resultierenden wässrigen Phase oder wässrigen Phasen, und
(e) optional Zuführung zumindest eines Teiles der aus Stufe (d) resultierenden wässrigen Phase zu einer biologischen Abwasseraufbereitung,
wobei die aus Stufe (a) beziehungsweise Stufe (b) resultierende wässrigen Phase (W1-res) keinem thermischen und/oder oxidativen Abbau unterzogen wird.

Die vorgenannten Aufarbeitungsstufen werden nachfolgend näher ausgeführt.

### Stufe (b)

In einer bevorzugten Ausführungsform erfolgt gemäß Stufe (b) die Entfernung organischer Bestandteile aus mindestens einem Teil der in Stufe (a) erhaltenen wässrigen Phase oder wässrigen Phasen durch Strippung.

Unter Strippen ist die Entfernung von bestimmten flüchtigen Bestandteilen aus Flüssigkeiten durch das Durchleiten von Gasen (Stickstoff, Wasserdampf, etc.) zu verstehen, wobei die genannten Bestandteile in die Gasphase übergeführt werden beziehungsweise mit der Gasphase aus der Flüssigkeit ausgetragen werden.

Vorzugsweise wird die Strippung im Rahmen der vorliegenden Erfindung mit Wasserdampf durchgeführt. Die Strippung erfolgt vorzugsweise in einer Strippkolonne, wobei die organischen Bestandteile, insbesondere Benzol und Nitrobenzol, über Kopf abgetrennt werden. Die Strippkolonne ist vorzugsweise eine röhrenförmige Einrichtung mit Einbauten zum intensiven Stoffaustausch von gasförmiger und flüssiger Phase. Vorzugsweise wird die Flüssigkeit im Gegenstrom, das heißt gegen die Fließrichtung des Gases, durch die Strippkolonne geleitet. Entsprechende Verfahren und Kolonnen sind dem Fachmann bekannt und z.B. in W. Meier, Sulzer, Kolonnen für Rektifikation und Absorption, in: Technische Rundschau Sulzer, 2 (1979), Seite 49 ff, beschrieben.

Bevorzugte Verfahren sind beispielsweise die Strippung in einer Kolonne, die vorzugsweise mit zufälligen Schüttungen aus Füllkörpern, mit strukturierten Packungen oder mit Stoffaustauschböden wie z.B. Siebböden, Glockenböden, Tunnelböden oder Thormannböden gefüllt ist. Vorzugsweise wird die Strippung gemäß Stufe (b) bei einem absoluten Druck von 0,1 bis 10 bar, insbesondere 1 bis 5 bar und einer Temperatur von 35 bis 180 °C, insbesondere bei 100 bis 160 °C durchgeführt.

Das im Rahmen von Stufe (b) anfallende, die aromatische Ausgangsverbindung und die aromatische Nitroverbindung sowie nicht-aromatische organische Verbindungen enthaltende Kondensat wird anschließend einem Phasenscheider zugeführt, wobei die organische Phase bevorzugt in die Wäsche in Stufe (a) zurückgeführt wird und die wässrige Phase erneut Stufe (b) zugeführt wird. In einer bevorzugten Ausführungsform wird der über Kopf der Strippkolonne erhaltene Wasserdampf einschließlich der organischen Bestandteile als Wärmeüberträger im Rahmen von Stufe (d) verwendet und das dabei anfallende Kondensat einem Phasenscheider zugeführt, wobei die organische Phase bevorzugt in die Wäsche in Stufe (a) zurückgeführt wird und die wässrige Phase vorzugsweise erneut Stufe (b) zugeführt wird. Diese bevorzugte Ausführungsform wird im Rahmen von Stufe (d) weiter erläutert.

Aus Sicherheitsgründen ist eine fehlerfreie Funktion von Stufe (b) wünschenswert. Eine Fehlfunktion der Strippkolonne kann beispielsweise durch redundante Sicherheitseinrichtungen überwacht werden.

Bevorzugt wird in Stufe (b) ein alkalisches Abwasser erhalten, das Benzol nur noch in Konzentrationen von höchstens 30 ppm, insbesondere höchstens 5 ppm, und Nitrobenzol in Konzentrationen von höchstens 50 ppm, insbesondere höchstens 20 ppm, enthält.

### Stufe (c)

Im Rahmen der vorliegenden Erfindung erfolgt im Rahmen von Stufe (c) die Entfernung organischer Verbindungen aus mindestens einem Teil der aus Stufe (a) beziehungsweise Stufe (b) resultierenden wässrigen Phase oder wässrigen Phasen durch thermischen und/oder oxidativen Abbau.

Die Entfernung organischer Verbindungen aus mindestens einem Teil der aus Stufe (a) beziehungsweise Stufe (b) resultierenden wässrigen Phase oder wässrigen Phasen durch thermischen Abbau wird nachfolgend als Thermolyse bezeichnet. Alternativ erfolgt der Abbau oxidativ, insbesondere durch Ozon (Ozonolyse).

Vorzugsweise wird in Stufe (c) das aus den Stufen (a) beziehungsweise (b) erhaltene Abwasser, das noch mit organischen Salzen der Nitrohydroxyaromaten beladen ist, unter Ausschluss von Sauerstoff auf Temperaturen von 150 bis 500 °C, vorzugsweise von 250 bis 350 °C, besonders bevorzugt 250 bis 300 °C unter Überdruck erhitzt. Es ist auch möglich, die Abwässer unter Inertgasatmosphäre bzw. unter einem Inertgas-Vordruck von beispielsweise 0,1 bis 100 bar zu erhitzen. Als Inertgase sind z.B. Stickstoff und/oder Argon geeignet. Je nach Temperatur und gegebenenfalls Inertgas-Vordruck stellen sich beim Erhitzen der Abwässer bevorzugt absolute Drücke im Bereich von 50 bis 350 bar, besonders bevorzugt 50 bis 200 bar, ganz besonders bevorzugt 70 bis 130 bar, ein. Die Erhitzung des alkalischen Abwassers und thermische Druckzersetzung der Nitrophenole erfolgt dabei üblicherweise für 5 bis 120 min, bevorzugt 20 bis 45 min.

Das Abwasser wird in Druckbehältern bei einer Temperatur von 150 °C bis 350 °C, vorzugsweise 250 °C bis 300 °C, einem Druck von 10 bar bis 200 bar, vorzugsweise 70 bar bis 150 bar und einem pH-Wert des Abwassers von 8 bis 14, vorzugsweise 9 bis 13 thermolysiert.

Als Druckbehälter können alle aus dem Stand der Technik bekannten Druckbehälter eingesetzt werden, die für die oben genannten Temperaturen und Drücke ausgelegt sind. Für eine kontinuierliche Verfahrensführung eignen sich beispielsweise Rohrreaktoren und in Kaskade geschaltete Autoklaven.

In einer bevorzugten Ausgestaltung wird das Abwasser mit einer Pumpe durch einen Wärmetauscher gefördert, in dem es z.B. auf 280 °C vorgewärmt wird. Anschließend wird das vorgewärmte Abwasser durch Direkteinspeisung von 100 bar Dampf oder durch eine indirekte Beheizung auf 300 °C erhitzt. Nach einer Verweilzeit von 20 min bis 60 min wird die Reaktionslösung im Gegenstrom mit dem Feed abgekühlt und entspannt.

Zur kontinuierlichen Ausgestaltung des Verfahrens benutzt man vorzugsweise einen Rohrreaktor, in welchem die Strömung der Flüssigkeit so eingestellt ist, dass eine Rückvermischung unterbleibt.

Vorzugsweise wird Stufe (c) als Thermolyse in Abwesenheit eines Inertgases bei einem absoluten Druck von 50 bis 350 bar und einer Temperatur von 150 bis 500 °C durchgeführt.

In einer alternativen Ausführungsform erfolgt die Entfernung organischer Verbindungen, insbesondere Nitrophenole, aus mindestens einem Teil der aus Stufe (a) beziehungsweise Stufe (b) resultierenden wässrigen Phase oder wässrigen Phasen durch oxidativen Abbau, bevorzugt durch Ozonolyse.

Verfahren zur Ozonolyse von Abwässern aus der Nitrierung von aromatischen Verbindungen sind dem Fachmann ebenfalls bekannt. Vorzugsweise erfolgt der oxidative Abbau durch Behandlung mit Ozon bei 20 bis 100 °C, einem Druck von 1,5 bis 10 bar und einem pH-Wert von 3 bis 12. Die Ozonolyse wird vorzugsweise kontinuierlich in einer Kaskade von Reaktoren, die im Gegenstrom geschaltet sind, durchgeführt. Auf diese Weise wird das Ozon so vollständig aus dem Gasstrom entfernt, dass man meist auf eine Restozonvernichtung verzichten kann. Entsprechende Verfahren sind insbesondere in der EP 0 378 994 A1 beschrieben, auf deren Inhalt hiermit in vollem Umfang Bezug genommen wird.

Nach Abschluss von Stufe (c) beträgt der Gehalt an Nitrophenolen im Abwasser vorzugsweise höchstens 100 ppm, insbesondere höchstens 30 ppm. Der Gehalt an Ammoniak im Abwasser, welches aus Stufe (c) resultiert, beträgt üblicherweise von 100 bis 3000 ppm, insbesondere von 500 bis 1500 ppm. Der Gehalt an Nitrat im Abwasser, welches aus Stufe (c) resultiert, beträgt üblicherweise von 5 bis 500 ppm, insbesondere von 20 bis 300 ppm. Der Gehalt an Nitrit im Abwasser, welches aus Stufe (c) resultiert, beträgt üblicherweise von 200 bis 10000 ppm, insbesondere von 500 bis 3000 ppm. Der Gehalt an organisch gebundenem Stickstoff (atomar berechnet) im Abwasser, welches aus Stufe (c) resultiert, beträgt üblicherweise von 5 bis 200 ppm, insbesondere von 5 bis 40 ppm.

### Stufe (d)

Gemäß der vorliegenden Erfindung erfolgt im Rahmen von Stufe (d) die destillative Abreicherung von Ammoniak aus der aus Stufe (b) beziehungsweise (c) resultierenden wässrigen Phase oder wässrigen Phasen. Die Destillation der wässrigen Phase(n) kann dabei nach an sich bekannten Verfahren erfolgen.

Vorzugsweise wird die Destillation gemäß Stufe (d) bei einem absoluten Druck von 0,1 bis 10 bar, insbesondere 1 bis 5 bar durchgeführt, wobei der genannte Druck am Kopf der Destillationsapparatur vorliegt.

Vorzugsweise wird die destillative Entfernung von Ammoniak gemäß Stufe (d) bei einer Temperatur von 80 bis 140 °C durchgeführt, wobei die genannte Temperatur am Kopf der Destillationsapparatur vorliegt.

Bevorzugt beträgt der Gehalt an Ammoniak in der wässrigen Phase in Anschluss an Stufe (d) höchstens 100 ppm, insbesondere höchstens 20 ppm, besonders bevorzugt höchstens 10 ppm. Die destillative Abreicherung von Ammoniak aus der aus Stufe (b) resultierenden wässrigen Phase(n) wird bevorzugt bei Temperaturen von 50 bis 160 °C und absoluten Drücken von 0,1 bis 10 bar, insbesondere 1 bis 5 bar realisiert.

Die destillative Abreicherung von Ammoniak kann in bekannten Apparaturen erfolgen. Die Verdampfung von Ammoniak erfolgt geeigneter weise in einer Destillationskolonne. Die Kolonne kann dabei mit dem Fachmann bekannten unstrukturierten Packungen wie z.B. zufälligen Schüttungen aus Füllkörpern, mit strukturierten Packungen oder mit Stoffaustauschböden wie z.B. Siebböden, Glockenböden, Tunnelböden oder Thormannböden gefüllt sein. In einer besonders bevorzugten Ausführungsform werden unstrukturierte oder strukturierte Packungen und Stoffaustauschböden miteinander kombiniert, so dass ein optimaler Trenneffekt erreicht wird..

Der Wärmeeintrag in die Destillationskolonne erfolgt vorzugsweise durch einen angekoppelten Verdampfer. Hierdurch lässt sich Stufe (d) in energetisch besonders günstiger Weise in das Verfahren integrieren.

Ein Verdampfer ist in der Verfahrenstechnik ein Apparat zur Umwandlung einer Flüssigkeit in ihren dampfförmigen Zustand. Zur Verdampfung der Flüssigkeit ist die Zuführung thermischer Energie erforderlich. Verdampfer bestehen deshalb in der Regel aus einer Oberfläche, durch die Wärme von einem Wärmeträger, vorzugsweise eine Flüsigkeit, auf die zu verdampfende Flüssigkeit übertragen wird. Im Rahmen der vorliegenden Erfindung bevorzugt sind Verdampfer, welche die erforderliche Wärme indirekt übertragen (kein direkter Kontakt zwischen Wärmeträger und zu verdampfende Flüssigkeit). Entsprechend Verdampfer sind dem Fachmann an sich bekannt. Geeignete Verdampfer sind insbesondere Naturumlaufverdampfer, Zwangsumlaufverdampfer, Kettle-Verdampfer, Dampfkessel, Fallfilmverdampfer und Dünnschichtverdampfer. Besonders geeignet sind Verdampfer auf Basis eines Rohrbündels. Besonders bevorzugt sind Fallfilmverdampfer.

In einer besonders bevorzugten Ausführungsform erfolgt die wärmetechnische Kopplung von Stufe (d) an mindestens eine vorausgehende Stufe, insbesondere Stufe (b).

Dabei wird die aus Stufe (b) resultierende Dampfphase vorzugsweise zur indirekten Wärmeübertragung in Stufe (d) verwendet. Besonders bevorzugt wird die aus Stufe (b) resultierende Dampfphase als Wärmeüberträger in einen Verdampfer im Rahmen von Stufe (d) eingeführt.

Vorzugsweise wird der Wärmeüberträger in Anschluss an die Stufe (d) zumindest teilweise in die Stufe (a) zurückgeführt. In einer besonders bevorzugten Ausführungsform wird der Wärmeüberträger in Anschluss an die Stufe (d) einer Phasentrennung unter Erhalt einer organischen Phase und einer wässrigen Phase unterzogen, wobei die erhaltene organische Phase in die Stufe (a) zurückgeführt wird. Vorzugsweise wird die erhaltene wässrige Phase Stufe (b) zugeführt.

Das durch das erfindungsgemäße Verfahren erhältliche, ganz oder teilweise von Ammoniak befreite Abwasser kann unmittelbar, d. h. ohne weitere Trennschritte, einer biologischen Abwasseraufbereitung, insbesondere einer Kläranlage, zugeführt werden. Das hierbei erhaltene Kopfprodukt enthaltend Ammoniak wird vorzugsweise nach dem Fachmann an sich bekannten Methoden kondensiert und vorzugsweise teilweise als Kondensatrücklauf in die Destillationskolonne im Rahmen von Stufe (d) zurückgeführt und teilweise einer weiteren Aufarbeitung, vorzugsweise einer Verbrennung, zugeführt. Nicht kondensierte Bestandteile können einer weiteren Abgasbehandlung zugeführt werden.

### Stufe (e)

Im Rahmen von Stufe (e) erfolgt vorzugsweise die Zuführung zumindest eines Teiles der aus Stufe (d) resultierenden wässrigen Phase zu einer biologischen Abwasseraufbereitungsstufe.

Entsprechende Verfahren zur biologischen Abwasseraufbereitung sind dem Fachmann an sich ebenfalls bekannt und beispielsweise in Ullmann's Encyclopedia of Industrial Chemistry 7th edition (Chapter "Waste Water"), 2005 Wiley-VCH Verlag GmbH & Co. KGaA, Weinheim ausführlich beschrieben.

### Beispiele

### Beispiel 1:

Stufe a1, Subschritt 1: 21 t/h rohes Nitrobenzol aus der Nitrierung von Benzol enthaltend 1000 Gew.-ppm Pikrinsäure und 2300 Gew.-ppm Dinitrophenol wurden mit 0,19 t/h 25%iger Natronlauge und der wässrigen Phase aus Stufe a1, Subschritt 2 versetzt und in einem Rührkessel bei 130 min⁻¹ intensiv vermischt.

Stufe a1, Subschritt 2: Nach anschließender Phasentrennung in einer nachfolgenden Phasentrennapparatur wird die organische Phase aus Stufe a1, Subschritt 1 mit 8 t/h mit NaOH alkalisch gestelltem Kondensat aus der Vakuumdestillation einer Schwefelsäureaufkonzentrierung und 0.001 Gew.% des Demulgators D1 bezogen auf die Gewichtsmenge Nitrobenzol versetzt und erneut intensiv bei 100 min⁻¹ vermischt. Nach der Phasentrennung wurde die wässrige Phase in Stufe a1, Subschritt 1 zurückgeführt. Die organische Phase wurde der Stufe a2 zugeführt. Die wässrige Phase aus Stufe a1, Subschritt 2 enthielt 6000 Gew.-ppm Nitrophenolate.

Stufe a2: Die organische Phase aus Stufe a1, Subschritt 2 wurde mit 6 t/h Abwasser aus einer Anilinproduktion (0,2 Gew.-% Mononitrobenzol und 200 ppm Ammoniak, pH 9) und ebenfalls 0,001 Gew.% Demulgator D1 versetzt und in einem Rührkessel bei 90 min⁻¹ vermischt. Nach anschließender Phasentrennung in einer nachfolgenden Phasentrennapparatur wurden 21 t/h Mononitrobenzol erhalten, welches frei von Nitrophenolen war (Gehalt unterhalb der Nachweisgrenze). Das Abwasser aus Stufe a2 enthielt weniger als 10 Gew.-ppm Nitrophenolate. Eine thermolytische oder ozonolytische Abwasseraufbereitung der wässrigen Phase kann unterbleiben.

Als Demulgator D1 wurde ein amphiphiles anionisches Copolymer enthaltend Olefin und Maleinsäureeinheiten (Na-Salz) als 25 Gew.-%ige wässrige Lösung mit einem pH-Wert nach DIN 19268 bei 23°C von 11, einer Jodzahl nach DIN EN 1557 bei 23°C von 2 und einem K-Wert nach ISO 1628-1 als 1 Gew.-%ige Lösung in dest. Wasser von 35 verwendet. Der Gehalt an Natriummaleat, berechnet als Maleinsäure, im Copolymer, bestimmt mittels HPLC, betrug 0,3 Gew.-%.

Der Wassergehalt der organischen Phasen betrug in Stufe a1, Subschritt 2 0,4 Gew.-% (bei 50°C), die Phasengrenzflächen waren in allen Fällen scharf.

### Vergleichsbeispiel 2:

Der gleiche Versuch wie im Beispiel wurde ohne Demulgator durchgeführt. Hierbei konnte keine scharfe Phasengrenzfläche in allen Stufen erreicht werden, der Wassergehalt stieg in den organischen Phasen der Waschstufe a1 auf bis zu 8,5% an.

## Patentansprüche

1. Verfahren zur Aufreinigung von rohen aromatischen Nitroverbindungen, die aus der Nitrierung von aromatischen Verbindungen stammen, umfassend die ein- oder mehrfache Durchführung der folgenden Waschstufe (a):
(a) Inkontaktbringen der rohen aromatischen Nitroverbindung (N-ein) mit einer wässrigen Phase (W-ein) und anschließende Phasentrennung unter Erhalt einer organischen Phase (N-res) und einer wässrigen Phase (W-res),
wobei in einer oder mehreren der Waschstufen (a) mindestens ein Demulgator (D) zugegen ist, der eine amphiphile Verbindung ist.

2. Verfahren nach Anspruch 1, wobei der Demulgator (D) in einer Menge von 1 bis 100 ppm bezogen auf die Gewichtsmenge der wässrigen Phase (W-ein) zugegen ist.

3. Verfahren nach einem oder mehreren der Ansprüche 1 bis 2, wobei der Demulgator (D) ein anionisches Copolymer ist.

4. Verfahren nach einem oder mehreren der Ansprüche 1 bis 3, wobei der Demulgator (D) ein Copolymer enthaltend Carboxylatgruppen ist.

5. Verfahren nach einem oder mehreren der Ansprüche 1 bis 4, wobei der Demulgator (D) ein Copolymer enthaltend Maleinsäureinheiten ist.

6. Verfahren nach einem oder mehreren der Ansprüche 1 bis 5, wobei der Demulgator (D) ein Copolymer enthaltend Maleinsäureinheiten und Olefineinheiten ist.

7. Verfahren nach einem oder mehreren der vorhergehenden Ansprüche, wobei Waschstufe (a) mindestens zweimal durchgeführt wird und die folgenden Waschstufen (a1) und (a2) umfasst, wobei jede der Stufen (a1) und (a2) ein- oder mehrfach hintereinander durchgeführt werden kann:
(a1) Inkontaktbringen der rohen aromatischen Nitroverbindung (N1-ein) mit einer ersten wässrigen Phase (W1-ein) umfassend mindestens eine Base (B) und anschließende Phasentrennung unter Erhalt einer organischen Phase (N1-res) und einer wässrigen Phase (W1-res); und anschließend
(a2) Inkontaktbringen der in Stufe (a1) erhaltenen organischen Phase (N1-res) mit einer zweiten wässrigen Phase (W2-ein) und anschließende Phasentrennung unter Erhalt einer aufgereinigten organischen Phase (N2-res) und mindestens einer wässrigen Phase (W2-res), wobei die eingesetzte wässrige Phase (W2-ein) einen pH-Wert von 6 bis 9 aufweist.

8. Verfahren nach Anspruch 7, wobei der pH-Wert der ersten wässrigen Phase (W1-ein) enthaltend mindestens eine Base (B) von 10 bis 14 beträgt.

9. Verfahren nach Anspruch 7 oder 8, wobei die erste wässrige Phase (W1-ein) Natriumhydroxid als Base (B) enthält.

10. Verfahren nach einem oder mehreren der Ansprüche 7 bis 9, wobei die Stufe (a1) in mindestens zwei Wiederholungen durchgeführt wird und die dabei eingesetzte wässrige Phase (W1-ein) im Rahmen der Waschstufe (a1) im Gegenstrom zur organischen Phase (N1-ein) geführt wird.

11. Verfahren nach oder mehreren der Ansprüche 7 bis 10, wobei die Stufe (a2) in zwei Wiederholungen durchgeführt wird und die dabei eingesetzte wässrige Phase (W2-ein) im Rahmen der Waschstufe (a2) im Gegenstrom zur organischen Phase (N2-ein) geführt wird.

12. Verfahren nach einem oder mehreren der Ansprüche 7 bis 11, wobei die wässrige Phase (W1-res) folgenden weiteren Aufarbeitungsstufen unterzogen wird:
(b) optional Entfernung organischer Bestandteile aus mindestens einem Teil der in Stufe (a) erhaltenen wässrigen Phase (W1-res) durch Strippung vorzugsweise mit Wasserdampf,
(c) Entfernung organischer Verbindungen aus mindestens einem Teil der aus Stufe (a) beziehungsweise Stufe (b) resultierenden wässrigen Phase (W1-res) durch thermischen und/oder oxidativen Abbau,
(d) destillative Abreicherung von Ammoniak aus zumindest einem Teil der aus Stufe (c) resultierenden wässrigen Phase, und
(e) optional Zuführung zumindest eines Teiles der aus Stufe (d) resultierenden wässrigen Phase zu einer biologischen Abwasseraufbereitung.

13. Verfahren nach einem oder mehreren der Ansprüche 7 bis 12, wobei die wässrige Phase (W2-res) folgenden weiteren Aufarbeitungsstufen unterzogen wird:
(b) optional Entfernung organischer Bestandteile aus mindestens einem Teil der in Stufe (a) erhaltenen wässrigen Phase (W2-res) durch Strippung vorzugsweise mit Wasserdampf,
(d) destillative Abreicherung von Ammoniak aus zumindest einem Teil der aus Stufe (c) resultierenden wässrigen Phase oder wässrigen Phasen, und
(e) optional Zuführung zumindest eines Teiles der aus Stufe (d) resultierenden wässrigen Phase zu einer biologischen Abwasseraufbereitung.

14. Verfahren nach einem oder mehreren der Ansprüche 7 bis 13, wobei als wässrige Phase (W2-ein) ein Abwasser aus einem anderen Herstellungsprozess verwendet wird, welches keine Nitrophenole enthält.

15. Verfahren nach einem oder mehreren der Ansprüche 7 bis 14, wobei als wässrige Phase (W2-ein) ein Abwasser eingesetzt wird, welches aus der Hydrierung von Mononitrobenzol stammt.

16. Verfahren nach einem oder mehreren der Ansprüche 7 bis 15, wobei bei Stufe (a1) ein Demulgator (D) zugegen ist.

17. Verfahren nach einem oder mehreren der Ansprüche 7 bis 16, wobei bei Stufe (a2) ein Demulgator (D) zugegen ist.

## Claims

1. A process for purifying crude aromatic nitro compounds which originate from the nitration of aromatic compounds, comprising the single or multiple performance of the following wash stage (a) :
(a) contacting the crude aromatic nitro compound (N-in) with an aqueous phase (W-in) and then separating the phases to obtain an organic phase (N-res) and an aqueous phase (W-res),
wherein at least one demulsifier (D) which is an amphiphilic compound is present in one or more of the wash stages (a).

2. The process according to claim 1, wherein the demulsifier (D) is present in an amount of 1 to 100 ppm, based on the weight of the aqueous phase (W-in).

3. The process according to one or more of claims 1 to 2, wherein the demulsifier (D) is an anionic copolymer.

4. The process according to one or more of claims 1 to 3, wherein the demulsifier (D) is a copolymer comprising carboxylate groups.

5. The process according to one or more of claims 1 to 4, wherein the demulsifier (D) is a copolymer comprising maleic acid units.

6. The process according to one or more of claims 1 to 5, wherein the demulsifier (D) is a copolymer comprising maleic acid units and olefin units.

7. The process according to one or more of the preceding claims, wherein wash stage (a) is performed at least twice and comprises the following wash stages (a1) and (a2), where each of stages (a1) and (a2) may be performed once or more than once in succession:
(a1) contacting the crude aromatic nitro compound (N1-in) with a first aqueous phase (W1-in) comprising at least one base (B) and then separating the phases to obtain an organic phase (N1-res) and an aqueous phase (W1-res); and then
(a2) contacting the organic phase (N1-res) obtained in stage (a1) with a second aqueous phase (W2-in) and then separating the phases to obtain a purified organic phase (N2-res) and at least one aqueous phase (W2-res), the aqueous phase used (W2-in) having a pH of 6 to 9.

8. The process according to claim 7, wherein the pH of the first aqueous phase (W1-in) comprising at least one base (B) is from 10 to 14.

9. The process according to claim 7 or 8, wherein the first aqueous phase (W1-in) comprises sodium hydroxide as the base (B).

10. The process according to one or more of claims 7 to 9, wherein stage (a1) is performed in at least two repetitions and the aqueous phase (W1-in) used is conducted in countercurrent to the organic phase (N1-in) in the course of wash stage (a1).

11. The process according to one or more of claims 7 to 10, wherein stage (a2) is performed in two repetitions and the aqueous phase (W2-in) used is conducted in countercurrent to the organic phase (N2-in) in the course of wash stage (a2).

12. The process according to one or more of claims 7 to 11, wherein the aqueous phase (W1-res) is subjected to the following further workup stages:
(b) optional removal of organic constituents from at least a portion of the aqueous phase (W1-res) obtained in stage (a) by stripping, preferably with steam,
(c) removal of organic compounds from at least a portion of the aqueous phase (W1-res) resulting from stage (a) or stage (b) by thermal and/or oxidative degradation,
(d) distillative depletion of ammonia from at least a portion of the aqueous phase resulting from stage (c), and
(e) optional supply of at least a portion of the aqueous phase resulting from stage (d) to a biological wastewater treatment.

13. The process according to one or more of claims 7 to 12, wherein the aqueous phase (W2-res) is subjected to the following further workup steps:
(b) optional removal of organic constituents from at least a portion of the aqueous phase (W2-res) obtained in stage (a) by stripping, preferably with steam,
(d) distillative depletion of ammonia from at least a portion of the aqueous phase(s) resulting from stage (c), and
(e) optional supply of at least a portion of the aqueous phase resulting from stage (d) to a biological wastewater treatment.

14. The process according to one or more of claims 7 to 13, wherein the aqueous phase (W2-in) used is a wastewater from a different production process which does not comprise any nitrophenols.

15. The process according to one or more of claims 7 to 14, wherein the aqueous phase (W2-in) used is a wastewater which is obtained from the hydrogenation of mononitrobenzene.

16. The process according to one or more of claims 7 to 15, wherein a demulsifier (D) is present in stage (a1).

17. The process according to one or more of claims 7 to 16, wherein a demulsifier (D) is present in stage (a2).

## Revendications

1. Procédé pour la purification de composés nitrés aromatiques qui proviennent de la nitration de composés aromatiques, comprenant l'exécution une ou plusieurs fois de l'étape de lavage (a) suivante :
(a) mise en contact du composé nitré aromatique brut (N-int) avec une phase aqueuse (W-int) et séparation de phases subséquente, avec obtention d'une phase organique (N-rés) et d'une phase aqueuse (W-rés),
dans lequel au moins un désémulsifiant (D), qui est un composé amphiphile, est ajouté dans une ou plusieurs des étapes de lavage (a).

2. Procédé selon la revendication 1, dans lequel le désémulsifiant (D) est ajouté en une quantité de 1 à 100 ppm par rapport au poids total de la phase aqueuse (W-int).

3. Procédé selon une ou plusieurs des revendications 1 et 2, dans lequel le désémulsifiant (D) est un copolymère anionique.

4. Procédé selon une ou plusieurs des revendications 1 à 3, dans lequel le désémulsifiant (D) est un copolymère contenant des groupes carboxylate.

5. Procédé selon une ou plusieurs des revendications 1 à 4, dans lequel le désémulsifiant (D) est un copolymère contenant des motifs acide maléique.

6. Procédé selon une ou plusieurs des revendications 1 à 5, dans lequel le désémulsifiant (D) est un copolymère contenant des motifs acide maléique et des motifs oléfiniques.

7. Procédé selon une ou plusieurs des revendications précédentes, dans lequel l'étape de lavage (a) est effectuée au moins deux fois et comprend les étapes de lavage (a1) et (a2) suivantes, chacune des étapes (a1) et (a2) pouvant être effectuée une ou plusieurs fois successivement :
(a1) mise en contact du composé nitré aromatique brut (N1-int) avec une première phase aqueuse (W1-int) comprenant au moins une base (B) et séparation de phases subséquente, avec obtention d'une phase organique (N1-rés) et d'une phase aqueuse (W1-rés), et ensuite
(a2) mise en contact de la phase organique (N1-rés) obtenue dans l'étape (a1) avec une deuxième phase aqueuse (W2-int) et séparation de phases subséquente avec obtention d'une phase organique purifiée (N2-rés) et d'au moins une phase aqueuse (W2-rés), la phase aqueuse introduite (W2-int) présentant un pH de 6 à 9.

8. Procédé selon la revendication 7, dans lequel le pH de la première phase aqueuse (W1-int) contenant une base (B) vaut de 10 à 14.

9. Procédé selon la revendication 7 ou 8, dans lequel la première phase aqueuse (W1-int) contient de l'hydroxyde de sodium en tant que base (B).

10. Procédé selon une ou plusieurs des revendications 7 à 9, dans lequel l'étape (a1) est effectuée en au moins deux répétitions et la phase aqueuse introduite dans cette étape (W1-int) est conduite dans le cadre de l'étape de lavage (a1) à contre-courant de la phase organique (N1-int).

11. Procédé selon une ou plusieurs des revendications 7 à 10, dans lequel l'étape (a2) est effectuée en deux répétitions et la phase aqueuse introduite dans cette étape (W2-int) est conduite dans le cadre de l'étape de lavage (a2) à contre-courant de la phase organique (N2-int).

12. Procédé selon une ou plusieurs des revendications 7 à 11, dans lequel on soumet la phase aqueuse (W1-rés) aux autres étapes de traitement final suivantes :
(b) optionnelle élimination de composants organiques d'au moins une partie de la phase aqueuse (W1-rés) obtenue dans l'étape (a), par entraînement de préférence à la vapeur d'eau,
(c) élimination de composés organiques d'au moins une partie de la phase aqueuse résultant (W1-rés) de l'étape (a) ou de l'étape (b), par dégradation par oxydation et/ou thermique,
(d) concentration d'ammoniac par distillation à partir d'au moins une partie de la phase aqueuse résultant de l'étape (c), et
(e) optionnel envoi d'au moins une partie de la phase aqueuse résultant de l'étape (d) à un traitement biologique d'eaux résiduaires.

13. Procédé selon une ou plusieurs des revendications 7 à 12, dans lequel on soumet la phase aqueuse (W2-rés) aux étapes supplémentaires de traitement final suivantes :
(b) optionnelle élimination de composants organiques d'au moins une partie de la phase aqueuse (W2-rés) obtenue dans l'étape (a), par entraînement de préférence à la vapeur d'eau,
(d) concentration d'ammoniac par distillation à partir d'au moins une partie de la phase aqueuse ou des phases aqueuses résultant de l'étape (c), et
(e) optionnel envoi d'au moins une partie de la phase aqueuse résultant de l'étape (d) à un traitement biologique d'eaux résiduaires.

14. Procédé selon une ou plusieurs des revendications 7 à 13, dans lequel on utilise comme phase aqueuse (W2-int) une eau résiduaire provenant d'un autre processus de production, qui ne contient pas de nitrophénols.

15. Procédé selon une ou plusieurs des revendications 7 à 14, dans lequel on utilise comme phase aqueuse (W2-int) une eau résiduaire qui provient de l'hydrogénation de mononitrobenzène.

16. Procédé selon une ou plusieurs des revendications 7 à 15, dans lequel un désémulsifiant (D) est ajouté lors de l'étape (a1).

17. Procédé selon une ou plusieurs des revendications 7 à 16, dans lequel un désémulsifiant (D) est ajouté lors de l'étape (a2).
